# EUROPÄISCHE PATENTANMELDUNG

(11) **EP 3 974 010 A1**
(43) Veröffentlichungstag der Anmeldung: **30.03.2022**
(21) Anmeldenummer: 20197755.0
(22) Anmeldetag: 23.09.2020
(51) Int. Cl.: A61M 3/00, A61M 5/31, A61M 5/50, A61M 35/00, A61B 17/00, A61M 5/32

(54) **MULTIFUNKTIONSSPRITZE**

(71) Anmelder: Hubertus Goller Gesellschaft m.b.H., 3400 Klosterneuburg (AT)
(72) Erfinder: GRUBER, Patrick, 1040 Wien (AT)
(74) Vertreter: Schwarz & Partner Patentanwälte GmbH

(57) **Zusammenfassung**

Die Erfindung betrifft eine Multifunktionsspritze (6), umfassend einen Spritzenzylinder (7) und einen axial im Spritzenzylinder (7) verschieblichen Spritzenkolben (9), wodurch ein vom Spritzenzylinder (7) und dem Spritzenkolben (9) eingeschlossenes Aufnahmevolumen gebildet ist, wobei der Spritzenzylinder (7) eine einstückig an diesem ausgebildete Spritzenmündung (8) zur Ausgabe eines im Aufnahmevolumen befindlichen Fluids aufweist, der Spritzenzylinder (7) am Übergang zur Spritzenmündung (8) einen zylinderförmigen Absatz (11) aufweist, welcher einen kleineren Durchmesser (da) als ein Körper (7a) des Spritzenzylinders (7) und einen größeren Durchmesser (da) als die Spritzenmündung (8) aufweist, wobei an der Spritzenmündung (8) für den Einsatz der Multifunktionsspritze (6) als Blasenspritze (16) ein Blasenspritzenaufsatz (14) mit Abbrechspitze (15) montierbar ist.

## Beschreibung

Die Erfindung betrifft eine Multifunktionsspritze, umfassend einen Spritzenzylinder und einen axial im Spritzenzylinder verschieblichen Spritzenkolben, wodurch ein vom Spritzenzylinder und dem Spritzenkolben eingeschlossenes Aufnahmevolumen gebildet ist, wobei der Spritzenzylinder eine einstückig an diesem ausgebildete Spritzenmündung zur Ausgabe eines im Aufnahmevolumen befindlichen Fluids aufweist.

Aus dem Stand der Technik sind Standardspritzen bekannt, bei denen ein Spritzenkolben axial verschieblich im Spritzenzylinder angeordnet ist. Standardgemäß weisen diese Spritzen eine Spritzenmündung auf, die gemäß der ISO-80369-7 als Luer-Kegel ausgebildet sind. Dadurch ist die Spritze kompatibel mit anderen genormten Anschlüssen.

Eine andere aus dem Stand der Technik bekannte Spritze wird von Cathejell als Blasenspritze mit Abbrechspitze vertrieben. Diese Spritze ist eine Ziehharmonikaspritze, d.h. kolbenlos mit einem zusammendrückbaren Spritzenzylinder, ausgebildet. Bekannt ist, dass die Ziehharmonikaspritze mit einem Gleitmittel befüllt wird und zum Verschließen der Ziehharmonikaspritze ein Blasenspritzenaufsatz auf ein offenes Ende der Ziehharmonikaspritze gesetzt wird. Der Blasenspritzenaufsatz ist durch die Abbrechspitze verschlossen und kann mit einer Hand geöffnet werden, indem die Abbrechspitze manuell vom Blasenspritzenaufsatz abgebrochen wird. Die Abbrechspitze ist derart im Blasenspritzenaufsatz befestigt, dass dieser nach dem Abbrechen der Abbrechspitze keine scharfen Kanten aufweist.

Die genannte Blasenspritze von Cathejell ist derart ausgebildet, dass der Spritzenkörper einerseits den zusammendrückbaren Ziehharmonikaabschnitt und andererseits einen steifen, scheibenartigen, vom Ziehharmonikaabschnitt beabstandeten Frontabschnitt umfasst, der vom Blasenspritzenaufsatz umgriffen ist. Da ein derartiger scheibenartiger Frontabschnitt bei Standardspritzen nicht vorhanden ist, ist ein unmittelbares Anbringen eines Blasenspritzenaufsatzes auf Standardspritzen nicht möglich.

Es ist die Aufgabe der Erfindung, eine handelsübliche Spritze mit einem Spritzenzylinder und einem axial im Spritzenzylinder verschieblichen Spritzenkolben konstruktiv derart umzugestalten, dass diese für mehrere Einsatzzwecke verwendet werden kann, insbesondere als Blasenspritze. Können beispielsweise für mehrere unterschiedliche Typen von Spritzen gleiche Spritzenzylinder eingesetzt werden, kann die Massenfertigung der Spritzenzylinder vereinfacht werden.

Diese Aufgabe wird gelöst durch eine Multifunktionsspritze, umfassend einen Spritzenzylinder und einen axial im Spritzenzylinder verschieblichen Spritzenkolben, wodurch ein vom Spritzenzylinder und dem Spritzenkolben eingeschlossenes Aufnahmevolumen gebildet ist, wobei der Spritzenzylinder eine einstückig an diesem ausgebildete Spritzenmündung zur Ausgabe eines im Aufnahmevolumen befindlichen Fluids aufweist, der Spritzenzylinder am Übergang zur Spritzenmündung einen zylinderförmigen Absatz aufweist, welcher einen kleineren Durchmesser als ein Körper des Spritzenzylinders und einen größeren Durchmesser als die Spritzenmündung aufweist, wobei an der Spritzenmündung für den Einsatz der Multifunktionsspritze als Blasenspritze ein Blasenspritzenaufsatz mit Abbrechspitze montierbar ist.

Bei der erfindungsgemäßen Multifunktionsspritze wird somit zusätzlich zur Spritzenmündung - und nicht alternativ dazu - ein Absatz zur Befestigung eines Blasenspritzenaufsatzes vorgesehen. Damit kann die Multifunktionsspritze unabhängig vom weiteren Einsatzzweck als Einheitsbauteil gefertigt werden, wodurch die Massenfertigung eines einzigen Bauteils sowohl für Blasenspritzen als auch durch Kappen verschlossene Spritzen ermöglicht wird. Ein weiterer Vorteil der erfindungsgemäßen Multifunktionsspritze besteht darin, dass durch den nachträglich montierbaren Blasenspritzenaufsatz mit Abbrechspitze die Spritze weiterhin von vorne durch die Spritzenmündung befüllt werden kann. Dies ist bei einer einstückig ausgeführten Spritze mit Abbrechspitze nicht gegeben. Eine derartige Spritze kann nur mehr von hinten befüllt werden und die Dichtung und die Kolbenstange der Spritze müssten aufwändiger nachträglich montiert werden. Auch die Ausführung von Dichtung und Kolbenstange für eine Befüllung von hinten ist in der Regel teurer.

Der erfindungsgemäße Absatz zwischen Spritzenzylinder und Spritzenmündung ermöglicht somit die Befestigung von diversen, nicht-standardgemäßen Verschlüssen wie insbesondere eines Blasenspritzenaufsatzes mit Abbrechspitze. Besonders ist bei dieser Lösung, dass weiterhin die Möglichkeit bestehen bleibt, die Spritzenmündung mit anderen Hilfsmitteln wie einer Kappe zu verschließen, um optional zur Verschlussmöglichkeit mit einem Blasenspritzenaufsatz eine Verschlussmöglichkeit mit abnehmbarer Kappe vorzusehen. Dadurch kann in der Massenproduktion ein einziger Spritzenkörper für eine Vielzahl von Verwendungszwecken gefertigt werden. Das Hindernis, für verschiedene Anwendungszwecke verschiedene Spritzenkörper fertigen zu müssen, fällt folglich weg. Die erfindungsgemäße Lösung des Absatzes zwischen Spritzenzylinder und Spritzenmündung hat überdies den Vorteil, dass der Außendurchmesser des Spritzenzylinders selbst bei einem aufgesetzten Blasenspritzenaufsatz nicht erhöht wird, wie dies bei der Blasenspritze von Cathejell der Fall ist. Mit der erfindungsgemäßen Lösung wird somit auch eine Kolbenspritze für die Einsatzmöglichkeit als Blasenspritze geschaffen, die einen standardgemäßen Außendurchmesser aufweist.

Besonders bevorzugt ist, wenn die Spritzenmündung eine Länge und einen Spitzendurchmesser aufweist, die von den Normmaßen eines in der ISO-80369-7 genormten Luer-Kegels abweichen, wobei bevorzugt die Länge der Spritzenmündung größer ist als die Länge eines in der ISO-80369-7 genormten Luer-Kegels und der Spitzendurchmesser der Spritzenmündung größer ist als der Spitzendurchmesser eines in der ISO-80369-7 genormten Luer-Kegels. Dadurch wird verhindert, dass die Spritze versehentlich vom Anwender an einem genormten Luer-Anschluss angewendet werden kann. Gemäß einer alternativen Ausführungsvariante kann die Spritzenmündung als Luer-Kegel gemäß ISO-80369-7 ausgeführt sein. Des Weiteren kann auch nur die Spritzenmündung 8 oder nur der Spritzendurchmesser ds von den Normmaßen eines in der ISO-80369-7 genormten Luer-Kegels abweichen.

Weiters ist bevorzugt, wenn der Spritzenzylinder ein einstückig gefertigtes Spritzgussteil ist. Dadurch wird einerseits die Herstellung des Spritzenzylinders vereinfacht und andererseits ist die Multifunktionsspritze auch weniger fehleranfällig. Lösbare Ausführungsformen, bei denen der Absatz nachträglich auf eine bereits bestehende Standardspritze aufgebracht wird, werden somit ausgeschlossen.

In einer besonders vorteilhaften Ausführungsform weist der genannte Absatz an seinem Außenumfang eine Rippe zur Befestigung des Blasenspritzenaufsatzes auf. Die Rippe ermöglicht, dass der Blasenspritzenaufsatz ohne den Einsatz weiterer Hilfsmittel auf dem Spritzenzylinder montiert werden kann. Dadurch ergibt sich ein besonders schneller Verschluss der Multifunktionssprite mit dem Blasenspritzenaufsatz.

Wird der Blasenspritzenaufsatz auf die Multifunktionsspritze aufgesetzt, ergibt sich erfindungsgemäß eine Blasenspritze. Um mit der Multifunktionsspritze kompatibel zu sein, weist der verwendete Blasenspritzenaufsatz einen der Abbrechspitze abgewandten Montageabschnitt auf, welcher einen Innendurchmesser hat, der im Wesentlichen dem Außendurchmesser des Absatzes der Multifunktionsspritze entspricht.

Um den Blasenspritzenaufsatz stabil und bevorzugt auch dauerhaft auf der Multifunktionsspritze zu befestigen, können insbesondere die zwei im Folgenden beschriebenen Ausführungsvarianten eingesetzt werden, die auch kombinierbar sind.

In der ersten Ausführungsvariante kann der Montageabschnitt des Blasenspritzenaufsatzes unlösbar auf dem Absatz montiert werden, bevorzugt durch Ultraschallschweißen, Laserschweißen oder Kleben. Bei dieser Lösung kommen zur Befestigung zusätzliche Hilfsmittel zum Einsatz, um den Blasenspritzenaufsatz besonders fest und dauerhaft mit der Multifunktionsspritze zu verbinden.

In der zweiten Ausführungsvariante weist der Montageabschnitt des Blasenspritzenaufsatzes an dessen Innenumfang eine Nut für den Eingriff der Rippe des Absatzes auf, sodass die Rippe mit der Nut in Eingriff gebracht werden kann. Die Lösbarkeit bzw. Unlösbarkeit der Rippe/Nut-Verbindung kann durch die Wahl der Materialien, Formen und Dimensionen beeinflusst werden. Zudem kann durch die Gestaltung der Rippe bzw. Nut vorgesehen werden, dass nur ein bestimmter Blasenspritzenaufsatz mit vorbestimmter Nut auf die Multifunktionsspritze aufgesetzt werden kann.

Weiters bevorzugt bestehen die Multifunktionsspritze und der Blasenspritzenaufsatz aus demselben Material, was insbesondere dann vorteilhaft ist, wenn die Anbringung des Blasenspritzenaufsatzes auf die Multifunktionsspritze durch Schweißen erfolgt.

Um eine kompakte Bauweise der Blasenspritze zu erzielen, kann zudem vorgesehen werden, dass der Montageabschnitt des Blasenspritzenaufsatzes einen Außendurchmesser aufweist, der maximal dem Durchmesser des Spritzenzylinders entspricht.

Um die Blasenspritze weiter zu verbessern, kann der Blasenspritzenaufsatz ein Innenprofil aufweisen, welches im Wesentlichen dem Außenprofil der Spritzenmündung entspricht, und wobei das Außenprofil der Spritzenmündung am Innenprofil des Blasenspritzenaufsatzes anliegt, wenn der Blasenspritzenaufsatz auf der Multifunktionsspritze montiert ist. Dadurch kann der Blasenspritzenaufsatz passfest auf die Spritzenmündung gesetzt werden, wodurch ein wackeln des Blasenspritzenaufsatzes verhindert wird. Ein weiterer Vorteil besteht darin, dass hierdurch kein in der Multifunktionsspritze befindliches Medium in Spalten oder Hohlräume der Konturen der erfindungsgemäßen Multifunktionsspritze dringen kann und als unbenutzte Restmenge bei der Anwendung in der Multifunktionsspritze verbleibt.

Solle die Multifunktionsspritze nicht als Blasenspritze eingesetzt werden, kann diese auch mit einer allgemeinen Kappe verschlossen werden, die nicht mit dem genannten Absatz interagiert. In dieser Variante wird die Spritze als Mehrfachverschlussspritze bezeichnet und umfasst die Multifunktionsspritze in einer der vorgenannten Ausführungsformen und eine Kappe, welche eine geringere Länge als die Spritzenmündung aufweist und die Spritzenmündung lösbar verschließt. Insbesondere bietet sich an, die Mehrfachverschlussspritze als Wundgelspritze zu vertreiben, d.h. die Mehrfachverschlussspritze wird z.B. mit Wundgel befüllt, bevor sie mit der Kappe verschlossen wird.

In einem weiteren Aspekt kann die Multifunktionsspritze zusammen mit einem noch nicht montierten Blasenspritzenaufsatz und/oder einer noch nicht montierten Kappe als Kit-of-Parts bereitgestellt werden. Dadurch kann vor dem Vertrieb rasch eine Umgestaltung der Sprite in eine Blasenspritze oder Mehrfachverschlussspritze vorgenommen werden.

Vorteilhafte und nicht einschränkende Ausführungsformen der Erfindung werden nachfolgend anhand der Zeichnungen näher erläutert.
Figur 1 zeigt eine Standardspritze nach dem Stand der Technik.
Figur 2 zeigt ein Detail einer erfindungsgemäßen Multifunktionsspritze.
Die Figuren 3a bis 3c zeigen die Komponenten der Multifunktionsspritze von Figur 2.
Die Figuren 3d und 3e zeigen zwei verschiedene Verschlussmöglichkeiten für die Multifunktionsspritze von Figur 2.
Figur 4 zeigt eine als Blasenspritze ausgeführte Multifunktionsspritze.
Die Figuren 5 und 6 zeigen jeweils ein Detail einer ersten Ausführungsvariante einer Blasenspritze.
Die Figuren 7 und 8 zeigen jeweils ein Detail einer zweiten Ausführungsvariante einer Blasenspritze.
Figur 9 zeigt eine als Mehrfachverschlussspritze ausgeführte Multifunktionsspritze.
Figur 10 zeigt ein Detail der Mehrfachverschlussspritze von Figur 9.

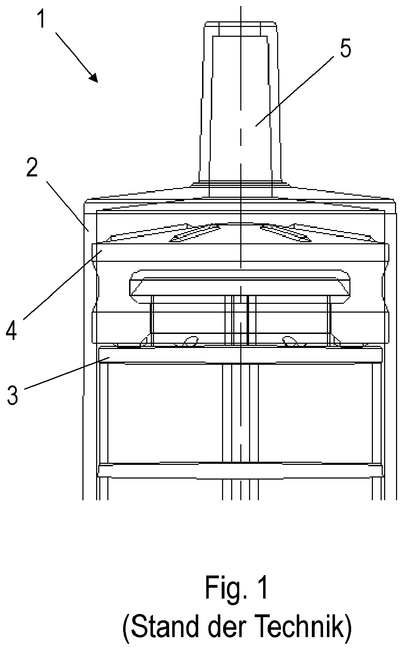

Figur 1 zeigt eine Standardspritze 1 gemäß dem Stand der Technik. Die Standardspritze 1 ist als Kolbenspritze ausgebildet und umfasst einen Spritzenzylinder 2 und einen axial im Spritzenzylinder 2 verschieblichen Spritzenkolben 3. Der Spritzenkolben 3 ist gegenüber dem Spritzenzylinder 2 mit einem Dichtelement 4 abgedichtet. Wird der Spritzenkolben 3 aus dem Spritzenzylinder 2 herausgezogen, so bildet sich ein nicht weiter dargestelltes Aufnahmevolumen zur Aufnahme eines Fluids zwischen dem Spritzenzylinder 2 und dem Spritzenkolben 3 aus. In bekannten Ausführungsformen ist das im Aufnahmevolumen aufgenommene Fluid beispielsweise ein Wundgel. Weitere in Standardspritzen 1 gemäß dem Stand der Technik verwendete Fluide sind dem Fachmann allgemein bekannt.

Zur Aufnahme und Ausgabe des Fluids in bzw. aus dem Aufnahmevolumen weist der Spritzenzylinder 2 eine Spritzenmündung 5 auf, die gemäß der ISO-80369-7 als genormter Luer-Kegel ausgeführt ist. Die Spritzenmündung 5 kann mit einer kompatiblen Kappe verschlossen werden.

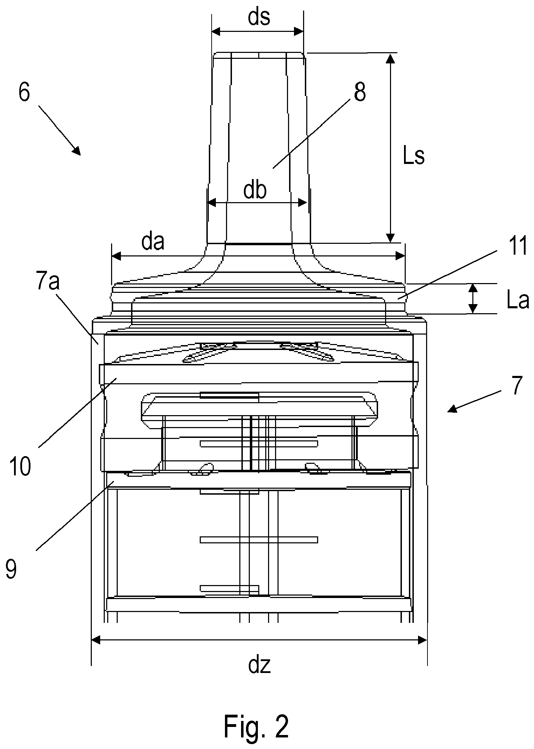

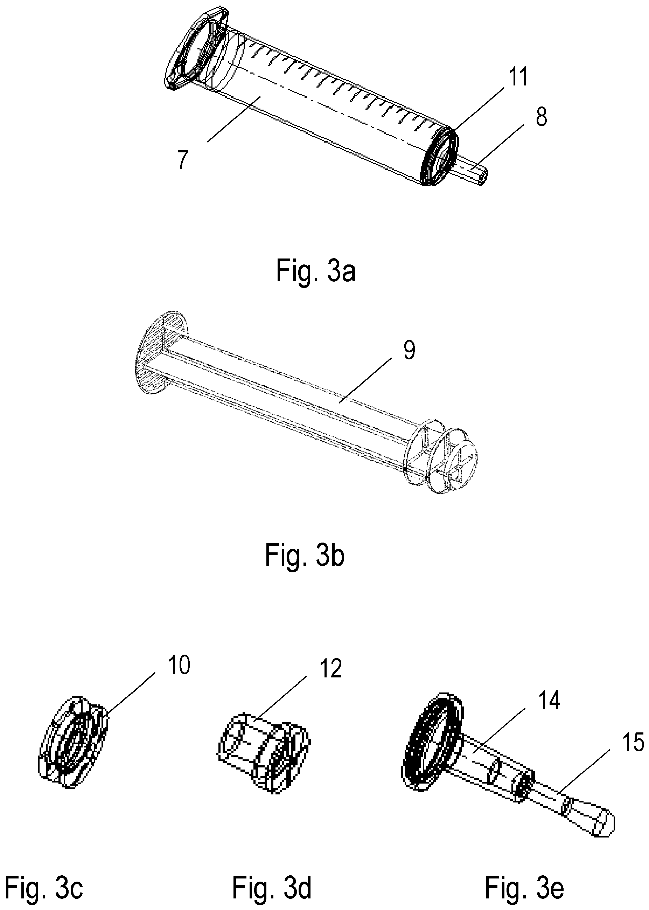

Figur 2 zeigt eine Multifunktionsspritze 6 gemäß der Erfindung. Auch die Multifunktionsspritze 6 umfasst einen Spritzenzylinder 7 (Figur 3a) mit Spritzenmündung 8 und einen axial im Spritzenzylinder 7 verschieblichen Spritzenkolben 9 (Figur 3b), welcher gegenüber dem Spritzenzylinder 7 mit einem Dichtelement 10 (Figur 3c) abgedichtet sein kann. Analog zur Standardspritze 1 aus Figur 1 kann der Spritzenkolben 9 aus dem Spritzenzylinder 7 herausgezogen werden, sodass sich ein nicht weiter dargestelltes Aufnahmevolumen zur Aufnahme eines Fluids zwischen dem Spritzenzylinder 7 und dem Spritzenkolben 9 ausbildet.

Während der Spritzenkolben 9 und das Dichtelement 10 gemäß dem Stand der Technik, d.h. wie in Figur 1, ausgebildet sein können, unterscheiden sich der Spritzenzylinder 7 der Multifunktionsspritze 6 vom Spritzenzylinder 2 der Standardspritze 1. Im Gegensatz zu der Ausführungsform von Figur 1 weist der Spritzenzylinder 7 der Multifunktionsspritze 6 von Figur 2 einen Absatz 11 am Übergang zur Spritzenmündung 8 auf, d.h. der Absatz 11 befindet sich zwischen einem Körper 7a des Spritzenzylinders 7 und der Spritzenmündung 8 des Spritzenzylinders 7. Als Körper 7a des Spritzenzylinders 7 wird jener Bereich bezeichnet, in dem der Spritzenkolben 9 aufgenommen ist.

Aus Figur 2 ist überdies ersichtlich, dass der Körper 7a des Spritzenzylinders 7 einen Außendurchmesser dz aufweist, der größer ist als der Außendurchmesser da des Absatzes 11. Die Spritzenmündung 8 ist in dieser Ausführungsform konisch und weist einen dem Absatz 11 zugewandten Basisdurchmesser db und einen dem Absatz 11 abgewandten Spitzendurchmesser ds auf, der kleiner ist als der Basisdurchmesser db. Es ergeben sich die Größenverhältnisse dz > da > db > ds. Allgemein wird der Basisdurchmesser db hierin auch als Durchmesser der Spritzenmündung 8 bezeichnet, auch wenn die Spritzenmündung 8 konisch ist. Die Multifunktionsspritze 6 weist zudem eine Länge Ls der Spritzenmündung und eine Höhe La des Absatzes 11 auf.

Gemäß einer beispielhaften Ausführungsform der erfindungsgemäßen Multifunktionsspritze 6 mit einem Volumen von 10 ml weist die Multifunktionsspritze 6 folgende Abmessungen auf:
Außendurchmesser dz = 17,24 mm
Außendurchmesser da = 15,00 mm
Basisdurchmesser db = 5,31 mm
Spitzendurchmesser ds = 4,72 mm
Länge Ls = 9,90 mm
Höhe La = 1,60 mm

Je nach Ausführungsform kann die Spritzenmündung 8 als Luer-Kegel ausgebildet sein, d.h. eine Länge Ls und einen Spitzendurchmesser ds aufweisen wie in der ISO-80369-7 genormt. Bei der hierin beschriebenen Multifunktionsspritze 6 wird in der Regel jedoch vorgesehen, dass die Spritzenmündung 8 eine Länge Ls und einen Spitzendurchmesser ds aufweist, die von den Normmaßen eines in der ISO-80369-7 genormten Luer-Kegels abweichen. Beispielsweise kann die Länge Ls der Spritzenmündung 8 größer als die Länge eines in der ISO-80369-7 genormten Luer-Kegels und der Spitzendurchmesser ds der Spritzenmündung 8 größer als der Spitzendurchmesser eines in der ISO-80369-7 genormten Luer-Kegels sein.

Gemäß einer alternativen Ausführungsvariante kann auch nur die Länge Ls der Spritzenmündung 8 oder nur der Spritzendurchmesser ds von den Normmaßen eines in der ISO-80369-7 genormten Luer-Kegels abweichen.

Spitzendurchmesser ds und Basisdurchmesser db sowie die Länge Ls sind abhängig von einer Ausführung als Luer-Kegel lt. ISO-80369-7 oder, wie oben ausgeführt, als Kegel, der größer als ein Luer-Kegel ist und daher nicht versehentlich mit einem genormten Luer-Anschluss verwendet werden kann.

Der Außendurchmesser dz ist abhängig vom Volumen der Multifunktionsspritze 6 und kann kleiner oder größer als oben angegeben sein.

Je nach Volumen der Multifunktionsspritze 6 und somit abhängig vom Außendurchmesser dz bzw. der Ausführung der Spritzenmündung 8 und dem Basisdurchmesser db muss dann der Außendurchmesser da für den Absatz 11 entsprechend angepasst werden.

Die Höhe La des Absatzes 11 wird ebenfalls an das Volumen der erfindungsgemäßen Multifunktionsspritze 6 angepasst. Dies erfolgt damit eine der Größe des zu montierenden Blasenspritzenaufsatzes 14 entsprechende, feste mechanische Verbindung ermöglicht wird. Vorzugsweise liegt die Höhe La im Bereich von 1mm bis 5mm.

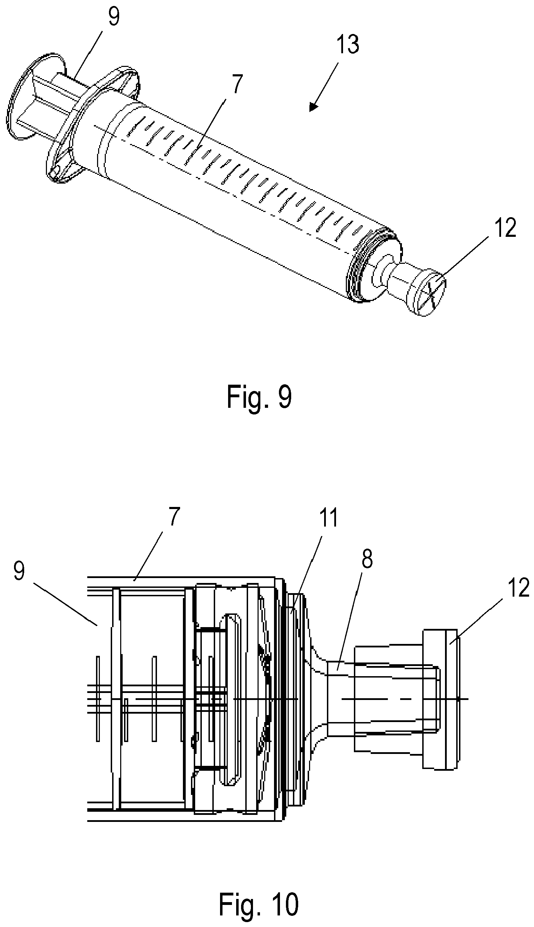

Die Multifunktionsspritze 6 kann aufgrund ihrer besonderen Ausbildung mit Absatz 11 und trotzdem vorhandener Spritzenmündung 8 für verschiedene Einsatzgebiete verwendet werden. So zeigt die Figur 3d eine Kappe 12, die auf die Spritzenmündung 8 aufgesetzt werden kann. Die Kappe 12 kann üblicherweise von der Spritzenmündung 8 genommen und danach wieder auf diese aufgesetzt werden, sodass die Multifunktionsspritze 6 wiederverschließbar ist. Aus diesem Grund wird die Spritze hierin als Mehrfachverschlussspritze 13 bezeichnet und kann beispielsweise als Wundgelspritze eingesetzt werden. Diese Verwendung wird unten anhand der Figuren 9 und 10 näher beschrieben.

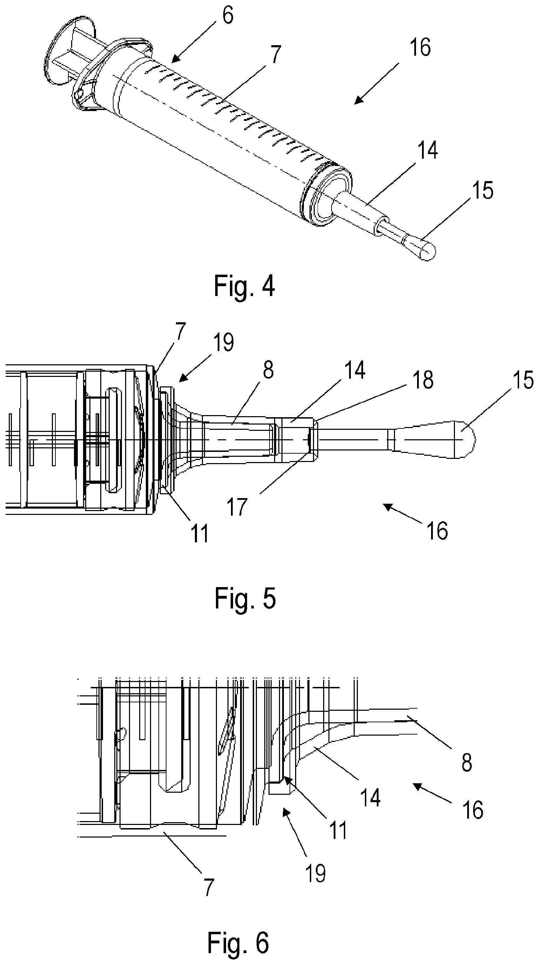

Figur 3e zeigt einen Blasenspritzenaufsatz 14 mit Abbrechspitze 15. Wie im Folgenden anhand der Figuren 4 bis 8 erläutert ist, kann die Multifunktionsspritze 6 mit aufgesetztem Blasenspritzenaufsatz 14 als Blasenspritze 16 eingesetzt werden. Der Blasenspritzenaufsatz 14 hat die vorteilhafte Eigenschaft, dass die Abbrechspitze 15 einhändig abgebrochen werden kann, wenn die Blasenspritze 16 in eine Hand genommen wird und die Abbrechspitze 15 auf eine unnachgiebige Oberfläche gedrückt wird. Dadurch kann die Blasenspritze 16 besonders hygienisch geöffnet werden. Ein weiterer Vorteil besteht darin, dass die erfindungsgemäße Blasenspritze 16 hierdurch nur mit einer Hand geöffnet werden kann.

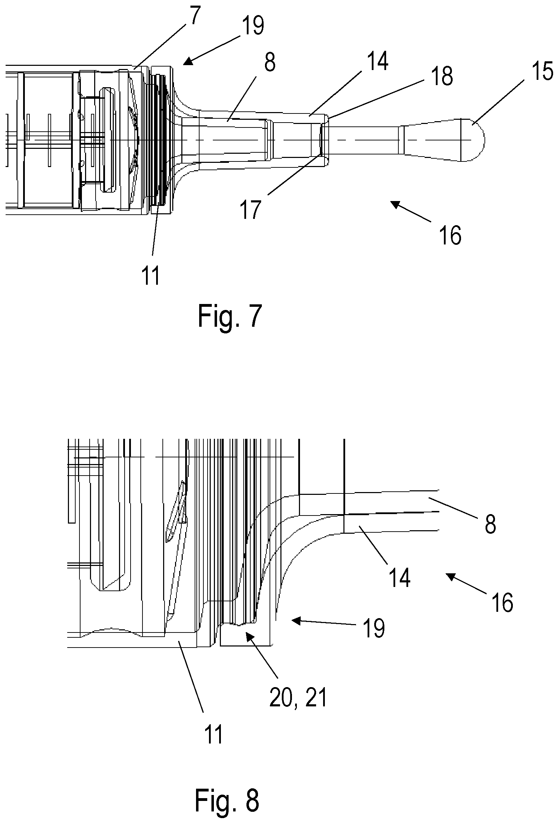

Vorgreifend auf die Figuren 5 und 7 ist überdies ersichtlich, dass die Abbrechspitze 15 vor dem Brechen im Inneren des Blasenspritzenaufsatzes 14 an einer Sollbruchstelle 17 befestigt ist, sodass nach dem Brechen keine außenliegende Bruchkante vorhanden ist. Dadurch kann die Öffnung 18 des Blasenspritzenaufsatzes 14 nach dem Abbrechen der Abbrechspitze 15 beispielsweise in oder an die Harnröhre geführt werden, ohne diese zu verletzen.

Figur 4 zeigt die Blasenspritze 16 umfassend die Multifunktionsspritze 6 und den Blasenspritzenaufsatz 14 mit Abbrechspitze 15. Es ist ersichtlich, dass die Spritzenmündung 8 vollständig im Blasenspritzenaufsatz 14 aufgenommen ist. Figur 5 zeigt im Detail, dass der Blasenspritzenaufsatz 14 eine Innenkontur aufweisen kann, die im Wesentlichen der Außenkontur der Spritzenmündung 8 entspricht. Dadurch kann der Blasenspritzenaufsatz 14 ohne Spiel auf die Spritzenmündung 8 aufgesetzt werden.

Aus Figur 6 ist ersichtlich, dass der Absatz 11 eine im Wesentlichen zylindrische Form mit glatter Oberfläche aufweist. Der Blasenspritzenaufsatz 14 weist am der Abbrechspitze 15 abgewandten Ende einen Montageabschnitt 19 auf, der in Umfangsrichtung und optional auch in der normal dazu verlaufenden Frontfläche auf dem Absatz 11 aufliegt. Der Montageabschnitt 19 hat somit einen Innendurchmesser, der im Wesentlichen dem Außendurchmesser da des Absatzes 11 der Multifunktionsspritze 6 entspricht.

Um den Blasenspritzenaufsatz 14 unlösbar mit dem Absatz 11 zu verbinden, können verschiedene Verbindungsmöglichkeiten vorgesehen werden. Beispielsweise kann der Blasenspritzenaufsatz 14 durch Ultraschallschweißen am Absatz 11 befestigt werden. Alternativ dazu kann die Befestigung durch Laserschweißen oder Kleben erfolgen. Insbesondere bei einem Schweißvorgang wird bevorzugt, wenn der Absatz 11 und der Blasenspritzenaufsatz 14 aus demselben Material, beispielsweise einem Polypropylen-Random-Copolymer wie PP Purell RP373R, gefertigt sind.

Die Figuren 7 und 8 zeigen jeweils eine Ausführungsform, bei der der Absatz 11 um den Umfang mit einer Rippe 20 versehen ist. Die Rippe 20 kann durch einen vollständig um den Umfang verlaufenden Steg oder durch eine oder mehrere Noppen gebildet sein. Komplementär hierzu weist der Blasenspritzenaufsatz 14 am Innenumfang eine Nut 21 auf, die der Form der Rippe 20 gegengleich entspricht. Alternativ hierzu könnte auch der Absatz 11 eine Nut aufweisen und der Blasenspritzenaufsatz 14 eine Rippe .

Der Blasenspritzenaufsatz 14 kann somit auf den Absatz 11 aufgesetzt werden, ohne dass weitere Befestigungsverfahren wie Schweißen oder Kleben vorgenommen werden müssten. Die Ausführungsvarianten von Figur 7 und Figur 5 könnten jedoch auch kombiniert werden, sodass der Absatz 11 und der Blasenspritzenaufsatz 14 einerseits durch eine Rille/NutVerbindung und andererseits durch eine Schweißverbindung und/oder Klebeverbindung verbunden werden.

Figur 9 zeigt die Multifunktionsspritze 6 als Mehrfachverschlussspritze 13 mit aufgesetzter Kappe 12. Figur 10 zeigt im Detail, dass die Kappe eine geringere Länge als die Spritzenmündung 8 hat, sodass der Verschluss mittels der Kappe 12 nicht mit dem Absatz 11 in Berührung kommt. Die Kappe 12 und die Spritzenmündung 8 sind komplementär ausgebildet. Zudem entspricht ein Verjüngungswinkel der Spritzenmündung 8 im Wesentlichen einem Verjüngungswinkel im Inneren der Kappe 12.

## Patentansprüche

1. Multifunktionsspritze (6), umfassend einen Spritzenzylinder (7) und einen axial im Spritzenzylinder (7) verschieblichen Spritzenkolben (9), wodurch ein vom Spritzenzylinder (7) und dem Spritzenkolben (9) eingeschlossenes Aufnahmevolumen gebildet ist,
wobei der Spritzenzylinder (7) eine einstückig an diesem ausgebildete Spritzenmündung (8) zur Ausgabe eines im Aufnahmevolumen befindlichen Fluids aufweist,
**dadurch gekennzeichnet, dass**
der Spritzenzylinder (7) am Übergang zur Spritzenmündung (8) einen zylinderförmigen Absatz (11) aufweist, welcher einen kleineren Durchmesser (da) als ein Körper (7a) des Spritzenzylinders (7) und einen größeren Durchmesser (da) als die Spritzenmündung (8) aufweist, wobei an der Spritzenmündung (8) für den Einsatz der Multifunktionsspritze (6) als Blasenspritze (16) ein Blasenspritzenaufsatz (14) mit Abbrechspitze (15) montierbar ist.

2. Multifunktionsspritze (6) nach Anspruch 1, wobei die Spritzenmündung (8) eine Länge (Ls) und einen Spitzendurchmesser (ds) aufweist, die von den Normmaßen eines in der ISO-80369-7 genormten Luer-Kegels abweichen, wobei bevorzugt die Länge (Ls) der Spritzenmündung (8) größer ist als die Länge eines in der ISO-80369-7 genormten Luer-Kegels und der Spitzendurchmesser (ds) der Spritzenmündung (8) größer ist als der Spitzendurchmesser eines in der ISO-80369-7 genormten Luer-Kegels.

3. Multifunktionsspritze (6) nach Anspruch 1, wobei die Spritzenmündung (8) eine Länge (Ls) und einen Spitzendurchmesser (ds) aufweist, die den Normmaßen eines in der ISO-80369-7 genormten Luer-Kegels entsprechen.

4. Multifunktionsspritze (6) nach einem der Ansprüche 1 bis 3, wobei der Spritzenzylinder (7) ein einstückig gefertigtes Spritzgussteil ist.

5. Multifunktionsspritze (6) nach einem der Ansprüche 1 bis 4, wobei der genannte Absatz (11) an seinem Außenumfang eine zumindest teilweise um den Umfang verlaufende Rippe (20) zur Befestigung des Blasenspritzenaufsatzes (14) aufweist.

6. Blasenspritze (16), umfassend eine Multifunktionsspritze (6) nach einem der Ansprüche 1 bis 5 und den Blasenspritzenaufsatz (14) mit Abbrechspitze (15), wobei der Blasenspritzenaufsatz (14) einen der Abbrechspitze (15) abgewandten Montageabschnitt (19) aufweist, welcher einen Innendurchmesser hat, der im Wesentlichen dem Außendurchmesser (da) des Absatzes (11) der Multifunktionsspritze (6) entspricht.

7. Blasenspritze (16) nach Anspruch 6, wobei der Montageabschnitt (19) des Blasenspritzenaufsatzes (14) unlösbar auf dem Absatz (11) montiert ist, bevorzugt durch Ultraschallschweißen, Laserschweißen oder Kleben.

8. Blasenspritze (16) nach Anspruch 6 oder 7, umfassend die Multifunktionsspritze (6) nach Anspruch 4, wobei der Montageabschnitt (19) des Blasenspritzenaufsatzes (14) an dessen Innenumfang eine Nut (21) für den Eingriff der Rippe (20) des Absatzes (11) aufweist.

9. Blasenspritze (16) nach einem der Ansprüche 6 bis 8, wobei die Multifunktionsspritze (6) und der Blasenspritzenaufsatz (14) aus demselben Material bestehen.

10. Blasenspritze (16) nach einem der Ansprüche 6 bis 9, wobei der Montageabschnitt (19) des Blasenspritzenaufsatzes (14) einen Außendurchmesser aufweist, der maximal dem Durchmesser des Körpers (7a) des Spritzenzylinders (7) entspricht.

11. Blasenspritze (16) nach einem der Ansprüche 6 bis 10, wobei der Blasenspritzenaufsatz (14) ein Innenprofil aufweist, welches im Wesentlichen dem Außenprofil der Spritzenmündung (8) entspricht, und wobei das Außenprofil der Spritzenmündung (8) am Innenprofil des Blasenspritzenaufsatzes (14) anliegt, wenn der Blasenspritzenaufsatz (14) auf der Multifunktionsspritze (16) montiert ist.

12. Mehrfachverschlussspritze (13), insbesondere Wundgelspritze, umfassend eine Multifunktionsspritze (6) nach einem der Ansprüche 1 bis 5 und eine Kappe (12), welche eine geringere Länge als die Spritzenmündung (8) aufweist und die Spritzenmündung (8) lösbar verschließt.

13. Kit-of-Parts, umfassend die Multifunktionsspritze (6) nach einem der Ansprüche 1 bis 5, den Blasenspritzenaufsatz (14) mit Abbrechspitze (15) und eine Kappe (12),
wobei der Blasenspritzenaufsatz (14) einen der Abbrechspitze (15) abgewandten Montageabschnitt (19) aufweist, welcher einen Innendurchmesser hat, der im Wesentlichen dem Außendurchmesser (da) des Absatzes (11) der Multifunktionsspritze (6) entspricht, und wobei die Kappe (12) eine geringere Länge als die Spritzenmündung (8) aufweist und die Spritzenmündung (8) lösbar verschließt.
